# EUROPÄISCHE PATENTSCHRIFT

(11) **EP 3 261 741 B1**
(45) Veröffentlichungstag und Bekanntmachung des Hinweises auf die Patenterteilung: **24.11.2021**
(21) Anmeldenummer: 16712219.1
(22) Anmeldetag: 29.02.2016
(51) Int. Cl.: B01D 15/18, A61K 31/35, C07D 311/80

(54) **CPC VERTEILUNGSCHROMATOGRAPHIE VON CANNABINOIDEN**
CPC PARTITION CHROMATOGRAPHY OF CANNABINOIDS
CHROMATOGRAPHIE DE PARTITION CPC DE CANNABINOÏDES

(30) Priorität: 27.02.2015 EP 15156901
(43) Veröffentlichungstag der Anmeldung: 03.01.2018
(73) Patentinhaber: Spectrum Therapeutics GmbH, 92318 Neumarkt (DE)
(72) Erfinder: RUTZ, Andreas, 91741 Dornhausen (DE)
(74) Vertreter: Mewburn Ellis LLP
(86) Internationale Anmeldenummer: PCT/EP2016/054250
(87) Internationale Veröffentlichungsnummer: WO 2016/135346

(56) Entgegenhaltungen:
- WO-A1-02/32420
- DE-A1-102005 028 937
- US-A1- 2014 243 405
- ARNO HAZEKAMP ET AL: "Preparative Isolation of Cannabinoids from Cannabis sativa by Centrifugal Partition Chromatography", JOURNAL OF LIQUID CHROMATOGRAPHY & RELATED TECHNOLOGIES, Bd. 27, Nr. 15, 11. Januar 2004 (2004-01-11), Seiten 2421-2439, XP055202081, ISSN: 1082-6076, DOI: 10.1081/JLC-200028170 in der Anmeldung erwähnt

## Beschreibung

Die Erfindung betrifft die Isolierung und Aufreinigung von Cannabinoiden aus Cannabis-Extrakt mittels *Centrifugal Partition Chromatography* (kurz: CPC).

Die CPC wird zur Gewinnung und Anreicherung von Pflanzeninhaltsstoffen aus Pflanzenextrakten im analytischen, semipräparativen sowie präparativen Maßstab eingesetzt. Die CPC ist ein flüssig-flüssig Chromatographieverfahren unter Verwendung eines zumeist zweiphasigen Lösungsmittelsystems.

Sie ermöglicht eine nahezu verlustfreie Separation hochkomplexer Substanzgemische aus Rohextrakten. Hersteller solcher Zentrifugal-Verteilung-Chromatographen zur Durchführung einer CPC ist z.B. Kromaton S.a.r.l (Annonay, FR) oder Armen Instrument Sas (Saint-Ave, FR).

Die CPC ist im Vergleich zur Flüssigchromatographie (HPLC) einfacher und zudem kostengünstiger, da Matrixeffekte sowie irreversible Adsorptionen an festen Phasen nicht vorkommen.

Bei der CPC Methode wird, wie bei gängigen flüssig-flüssigchromatographischen Methoden, wie zum Beispiel der High Speed Countercurrent Chromatography (HSCCC), ein 2-phasiges Lösungsmittelgemisch eingesetzt. Es kann wahlweise die obere oder die untere Phase als stationäre Phase verwendet werden. Anders jedoch als bei der HSCCC wird bei der CPC nicht mit einer Kapillarspule, sondern mit einem mit mehreren hundert Trennkammern versehenen Rotor gearbeitet. In diesen direkt hintereinander geschalteten Kammern findet die Verteilung der im Pflanzenextrakt enthaltenen Substanzen zwischen mobiler und stationärer Phase statt.

Das System wird während der Trennung in schnelle Rotation versetzt (bis zu 2.500 rpm). Dadurch wird zum einen, je nach Durchflußrichtung, die gewünschte Phase im Rotor der CPC zurückgehalten und zum anderen die Trennung der beiden Phasen durch die Zentrifugalkraft beschleunigt. Dies ermöglicht die Verwendung großer Flußgeschwindigkeiten und folglich den Durchsatz großer Substanzmengen in kurzer Zeit, so dass eine präparative Anwendung dieser Trenntechnik möglich ist.

Der Verteilungskoeffizient K der jeweils gewünschten Substanz zwischen den beiden Phasen sollte im Bereich zwischen 0,7 und 4,5 liegen. Bei kleinerem K eluiert die Substanz zu schnell, so dass keinerlei Trennung erfolgen kann. Bei höherem K hingegen wird die Retentionszeit für die schnelle Aufreinigung großer Mengen eines Pflanzenextrakts zu lang.

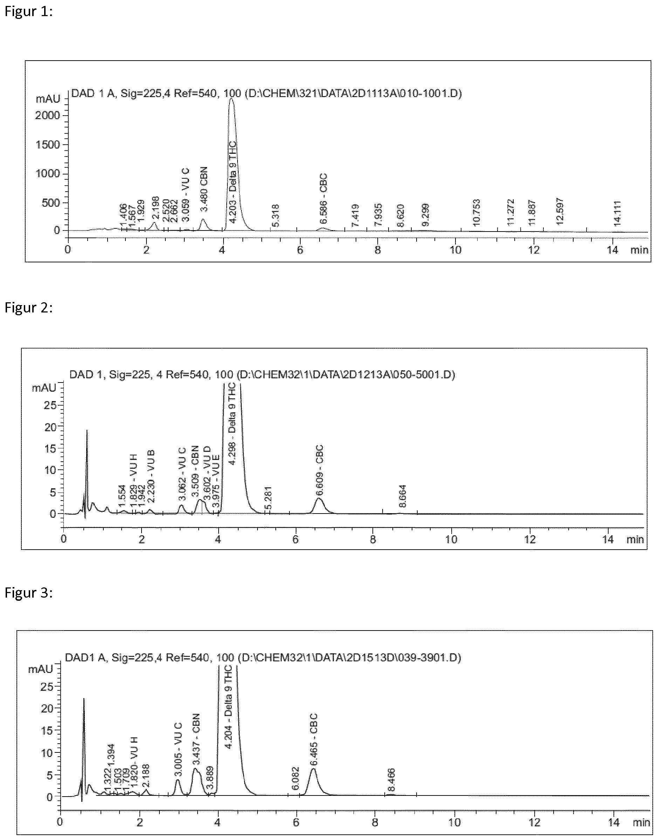

Im Stand der Technik ist beschrieben, dass Cannabinoide mittels CO₂ Extraktion aus Cannabis-Extrakten erhalten werden können (DE10051427C1). Dennoch werden die Cannabinoide, wie z.B. THC (A, "Dronabinol") oder CBD (B, Cannabidiol) nicht in absoluter Reinheit erhalten. 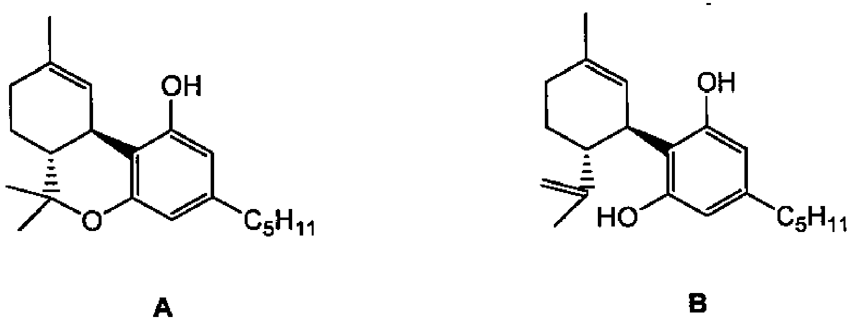 Abb. 1: Dronabinol (A) ((6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹THC)), Cannabidiol (CBD) (B) ((2-[1R-3-Methyl-6-(1-methylethenyl]-2-cyclohexen-1-yl]-5-pentyl-1,3-benzoldiol))

Die chromatographische Trennung und präparative Aufreinigung von Cannabinoiden stellt nach wie vor eine Herausforderung dar, insbesondere die Gewinnung von Cannabinoiden, vorzugsweise Δ⁹THC und CBD in hoher Reinheit von mehr als 95 %.

Im Stand der Technik beschreiben Hazekamp, A., 2007, Doctoral thesis, Leiden University und Arno Hazekamp, Ruud Simons, Anja Peltenburg-Looman, Melvin Sengers, Rianne van Zweden, Robert Verpoorte, Preparative isolation of cannabinoids from Cannabis sativa by centrifugal partition chromatography, J. Liq. Chrom. Rel. Technol. 2004, 27(15): 2421-2439, die präparative Gewinnung von Cannabinoiden mittels CPC. Allerdings wird im Stand der Technik ein Zwei-Phasen System auf der Basis von Hexan verwendet und eine Reinheit von max. 93,1 % erreicht. Zudem werden geringe Ausbeuten erreicht. Ferner ist Hexan ein Neurotoxin der Lösungsmittelklasse 2 (ICH Guidelines).

Es stellt sich somit die Aufgabe, ein verbessertes Verfahren für die Trennung und/oder Reinigung von Cannabinoiden aus Cannabis Pflanzenextrakten bereitzustellen, welches die vorliegenden Nachteile zumindest teilweise beseitigt und insbesondere in der Lage ist, bei vielen Anwendungen eine höhere Ausbeute und Reinheit an Cannabinoiden, insbesondere CBD und / oder THC präparativ bereitzustellen.

Die Aufgabe wird durch ein Verfahren nach einem der Patentansprüche gelöst.

Demgemäß wird ein Verfahren zur Trennung und/oder Reinigung von Cannabinoiden aus Cannabis-Extrakt bereitgestellt. Das Verfahren enthält mindestens einen Flüssig-flüssig-Verteilungschromatographieschritt, umfassend eine erste Lösungsmittelphase und eine zweite nicht mischbare flüssige Phase, wobei die erste Lösungsmittelphase Cyclohexan, Heptan, oder Octan ist und durch Zentrifugalkraft stationär gehalten wird, und 1 % bis 15 % (v/v) t-Butylmethylether der ersten Lösungsmittelphase und/oder der zweiten nicht mischbaren flüssigen Phase zugesetzt sind. n-Heptan ist jedoch erfindungsgemäß als die erste Lösungsmittelphase bevorzugt.

Die Dichte als auch die Viskosität von Cyclohexan, n-Heptan, iso-Octan sind größer als die Dichte/Viskosität von n-Hexan, so dass z.B. gegenüber der zweiten mobilen Phase wie z.B. Acetonitril ein stabileres Zweiphasensystem hergestellt wird, so dass eine bessere Retention der stationären Phase ermöglicht und folglich eine erhöhte Trennleistung erreicht wird, so dass gegenüber n-Hexan immer eine höhere Reinheit und Ausbeute erreicht wird; mit Reinheit von über 95 %, gar 99 %.

Das erfindungsgemäße Verfahren und/oder die erfindungsgemäße Verwendung zeigen vorteilhaft einen geringen Eluentenverbrauch neben keinen Ablagerungen an einer stationären Phase, so dass keine Regeneration der stationären Phase, bzw. Aufbereitung oder Entfernung von störenden Komponenten vonnöten ist. Die Produktwiederfindung ist nahezu quantitativ, da stationäre und mobile Phase einfach vertauscht werden können. Zudem wird die stationäre Phase bei jedem Lauf vollständig erneuert und kann durch Destillation einfach aufgereinigt bzw. erneuert werden. Die Reinheit der isolierten Cannabinoide ist oftmals so hoch, dass weitere chromatographische Aufreinigungsschritte entfallen können. Das Verfahren ist einfach ebenfalls im großtechnischen Maßstab durchführbar. Die Ausbeute an Cannabinoiden ist gegenüber dem Stand der Technik oftmals stark erhöht, wie im Folgenden noch genauer ausgeführt wird. Die Anzahl an benötigten Prozessstufen kann gegenüber alternativen Techniken meist deutlich vermindert werden. Besonders vorteilhaft kann die Gewinnung von Cannabinoiden aus beliebigen Cannabispflanzen und deren Extrakte erfolgen. Dies umfasst solche Cannabis Extrakte aus Cannabispflanzen (*Cannabis sativa, Cannabis indica, Cannabis ruderalis*), wie Hanf, Industriehanf, Nutzhanf, Drogenhanf, Faserhanf u.a..

Erfindungsgemäß ist für Cannabinoide die vorteilhafte Verwendung der beiden nicht miteinander mischbaren Lösungsmittel Acetonitril und Heptan bei einem Fluß von 50 bis 600 mL pro min. vorgesehen, vorzugsweise bei einem Fluss von 200 bis 300 mL pro min. während der Trennung und max. Fluss beim Spülen, bei einer Umdrehungszahl von 50 bis 1.500 rpm, vorzugsweise einer Umdrehungszahl von 900 bis 1.100 rpm während der Trennung. Weiterhin ist bevorzugt die Verwendung der oberen Phase als stationäre Phase.

In dem Verfahren der Erfindung ist der ersten Lösungsmittelphase und/oder der zweiten nicht mischbaren flüssigen Phase t-Butylmethylether (TBME) zu einem Anteil von 1 bis 15 % (v/v), und vorzugsweise 9 bis 13 % (v/v), zugesetzt.

Die zweite nicht mischbare flüssige Phase kann auch solche Lösungsmittel umfassen, wie Methanol, Ethylacetat oder Wasser, welche ggfs. mit 1-15 % t-Butylmethylether (TBME) versetzt sind.

Beispiele von Lösungsmittelsystemen zur Verwendung in dem Verfahren der Erfindung, in welchem TBME der stationären und/oder mobilen Phase zugesetzt ist, sind nicht abschließend (stationäre Phase/mobile Phase):
n-Heptan/Ethylacetat/Acetonitril,
n-Heptan/Ethylacetat/t-Butylmethylether/ Acetonitril,
n-Heptan/Ethylacetat/Methanol/Wasser,
n-Heptan/Methanol/Wasser,
n-Heptan/Ethanol/Wasser,
n-Heptan/Aceton/Wasser,
n-Heptan/Methanol/Acetonitril,
n-Heptan/Ethanol/Acetonitril,
n-Heptan/Aceton/Acetonitril,
n-Heptan/Chloroform/Acetonitril,
n-Heptan/Chloroform/Methanol,
n-Heptan/Methanol,
n-Heptan/Ethanol/Methanol,
n-Heptan/n-Butanol/Acetonitril,
n-Heptan/2-Propanol/Wasser,
n-Heptan/n-Propanol/Wasser,
n-Heptan/2-Propanol/Acetonitril,
n-Heptan/n-Propanol/Acetonitril,
n-Heptan/Dichloromethan/Acetonitril,
n-Heptan/Dichloromethan/Methanol,
n-Heptan/Tetrahydrofuran/Acetonitril,
n-Heptan/Benzotrifluorid/Acetonitril,
Cyclohexan/Methanol/Wasser,
Cyclohexan/Methanol/Acetonitril,
Cyclohexan/t-Butylmethylether/Wasser,
Cyclohexan/Acetonitril/Wasser,
Isooctan/Methanol,
Isooctan/Methanol/Wasser,
Isooctan/Ethylacetat/Methanol/Wasser.

Jedoch ist erfindungsgemäß Actetonitril, ggfs. versetzt mit 1-15 % t-Butylmethylether (TBME) besonders bevorzugt für die Verwendung als die zweite nicht mischbare flüssige Phase.

Weiterhin kann vorgesehen sein, dass das erfindungsgemäße Verfahren ein oder mehrfach durchlaufen wird (siehe z.B. Beispiel 1).

Die vorgenommene Auswahl dieser Lösungsmittel erlaubt eine Gewinnung von Cannabinoiden, vorzugsweise Δ⁹THC und CBD in hoher Reinheit von mehr als 95 %.

Daher stellen die Verfahren der Erfindung einen Cannabis-Extrakt bereit, wobei die Dronabinol Reinheit mehr als 95 %, insbesondere 99,6 % beträgt.

Daher stellen die Verfahren der Erfindung einen Cannabis-Extrakt bereit, wobei die Cannabidiol (CBD) Reinheit mehr als 95 %, insbesondere 99,3 % beträgt.

Bei Verwendung eines 12.500 mL fassenden CPC-Rotors ist zudem vorteilhaft eine Aufreinigung von bis zu 100 g Extrakt pro Lauf möglich. Pro Trennung werden inklusive Vorbereitungs- und Spülzeiten vorteilhaft lediglich 120 bis 160 min benötigt. Die Cannabinoide enthaltenden Fraktionen werden von den organischen Lösungsmitteln befreit. Nach Einengen im Vakuum erhält man Extrakte mit einem Massengehalt an gewünschtem Cannabinoid von bis zu 70-99 %, vorzugsweise mehr als 95 %.

Im Rahmen dieser Erfindung, werden unter Cannabinoiden insbesondere die folgenden Stoffe verstanden:
*Cannabigerol-artige (CBG):* Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃), Cannabigerolsäure A ((E)-CBGA-C₅ A), Cannabigerolsäure A Monomethylether ((E)-CBGAM-C₅ A), Cannabigerovarinsäure A ((E)-CBGVA-C₃ A) ;
*Cannabichromen-artige (CBC):* Cannabichromen (CBC-C₅), Cannabichromensäure A (CBCA-C₅ A), Cannabichromevarin (CBCV-C₃), Cannabichromevarinsäure A (CBCVA-C3 A);
*Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃);
*Cannabinodiol-artige (CBND):* Cannabinodiol (CBND-C₅), Cannabinodivarin (CBND-C₃);
*Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A (Δ9-THCVA-C₃ A), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅), (-)-Δ8-trans-(6aR,10aR)-Tetrahydrocannabinolsäure A (Δ8-THCA-C₅ A); (-)-(6aS,10aR)-Δ9-Tetrahydrocannabinol ((-)-cis-Δ9-THC-C₅);
*Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A (CBNA-C₅ A), Cannabinolmethylether (CBNM-C₅)
*Cannabitriol-artige (CBT):* (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((±)-cis-CBT-C₅), (-)-(9R,10R)-trans[10-O-Ethyl-cannabitriol] ((-)-trans-CBT-OEt-C₅), (±)-(9R,10R/9S,10S)-Cannabitriol-C₃ ((±)-t-rans-CBT-C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
*Cannabielsoin-artige (CBE) :* (5aS,6S,9R,9aR)- C₅-Cannabielsoin (CBE-C₅), (5aS, 6S, 9R, 9aR) -C₃-Cannabielsoin (CBE-C₃), (5aS,6S,9R,9aR)-Cannabielsoinsäure A (CBEA-C₅ A), (5aS,6S,9R,9aR)-Cannabielsoinsäure B (CBEA-C₅ B), (5aS,6S,9R,9aR)-C3-Cannabielsoinsäure B (CBEA-C₃ B), Cannabiglendol-C3 (OH-iso-HHCV-C₃), Dehydrocannabifuran (DCBF-C₅), Cannabifuran (CBF-C₅);
*Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-A7-trans-(1R,3R,6R)-Isotetrahydrocannabivarin;
*Cannabicyclol-artige (CBL):* (±)-(1aS,3aR,8bR,8cR)-Cannabicyclol (CBL-C₅), (±)-(1aS,3aR,8bR,8cR)-Cannabicyclolsäure A (CBLA-C₅ A), (±)-(1aS,3aR,8bR,8cR)-Cannabicyclovarin (CBLV-C₃);
*Cannabicitran-artige (CBT):* Cannabicitran (CBT-C₅);
*Cannabichromanon-artige (CBCN):* Cannabichromanon (CBCN-C₅), Cannabichromanon-C3 (CBCN-C₃), Cannabicoumaronon (CBCON-C₅).

Besonders bevorzugt sind jedoch Cannabidiol (CBD-C₅) und Δ9-Tetrahydrocannabinol (Δ9-THC-C₅).

Unter dem Begriff "Flüssig-flüssig-Verteilungschromatographieschritt" im Sinne der vorliegenden Erfindung wird insbesondere eine Chromatographie verstanden, bei der wie folgt vorgegangen wird: Eine bestimmte Menge eines Substanzgemisches wird mit einer flüssigen mobilen Phase durch eine stationär gehaltene Phase geleitet, wobei diese durch eine Zentrifugalkraft stationär gehalten wird. In einer weiteren bevorzugten Ausführungsform kann die Flüssig-flüssig-Verteilungschromatographie kontinuierlich durchgeführt werden. Hierzu werden die beiden Phasen im Gegenstrom geführt und eine kontinuierliche Abtrennung statt eines zeitversetzten (diskontinuierlichen) Austritts wird erreicht (siehe z.B. Yin, Lianhong; Li, Yingnan; Lu, Binan; Jia, Yujie; Peng, Jinyong, Trends in Counter-Current Chromatography: Applications to Natural Products Purification Separation and Purification Reviews (2010), 39(1-2), 33-62).

Entsprechend ihrer unterschiedlich starken Wechselwirkungen mit der stationären Phase treten die Substanzen kontinuierlich oder diskontinuierlich aus und können getrennt werden. Während jedoch bei der Flüssigchromatographie die stationäre Phase aus einem gepackten Festbett in einer Säule besteht, handelt es sich bei der Flüssig-flüssig-Verteilungschromatographie um eine zweite nicht mischbare flüssige Phase, die durch geeignete Vorrichtungen, wie z. B. einen Rotor durch Zentrifugalkraft stationär gehalten wird, insbesondere mittels einem entsprechenden Zentrifugal-Verteilung Chromatograph (supra).

"Cannabis Extrakt" im Sinne dieser Erfindung bedeutet ein beliebig aufgearbeiteter Extrakt aus einer Cannabispflanze oder Hanfpflanze, welche Cannabinoide enthält. Der Extrakt kann ein Primärextrakt, oder teilaufgearbeiteter Extrakt sein. Die Herstellung von Cannabis Extrakten ist im Stand der Technik hinreichend beschrieben. Geeignete Cannabispflanzen oder (Faser)hanfpflanzen sind solche wie Drogenhanf, Faserhanf.

In einer weiteren bevorzugten Ausführungsform kann mindestens eine präparative Säule (Festphase, wie z.B. Kieselgel) vor- oder nachgeschaltet sein (Beispiel 2).

### Beispiele und Figuren:

Diese Beispiele und Figuren dienen ausschließlich zur Erläuterung der Erfindung, ohne die Erfindung auf diese Beispiele zu beschränken.

### Beispiel 1: Cannabinoide aus Hanf

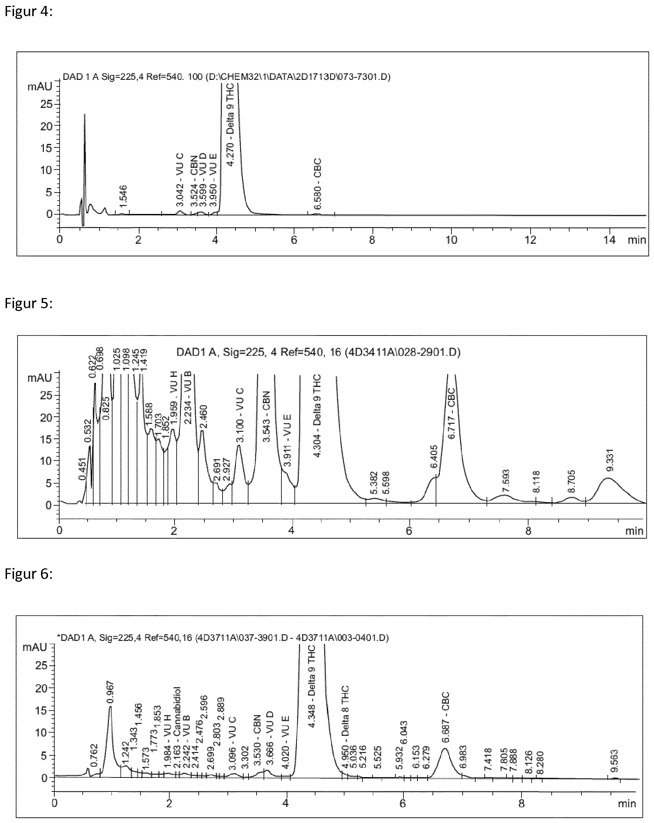

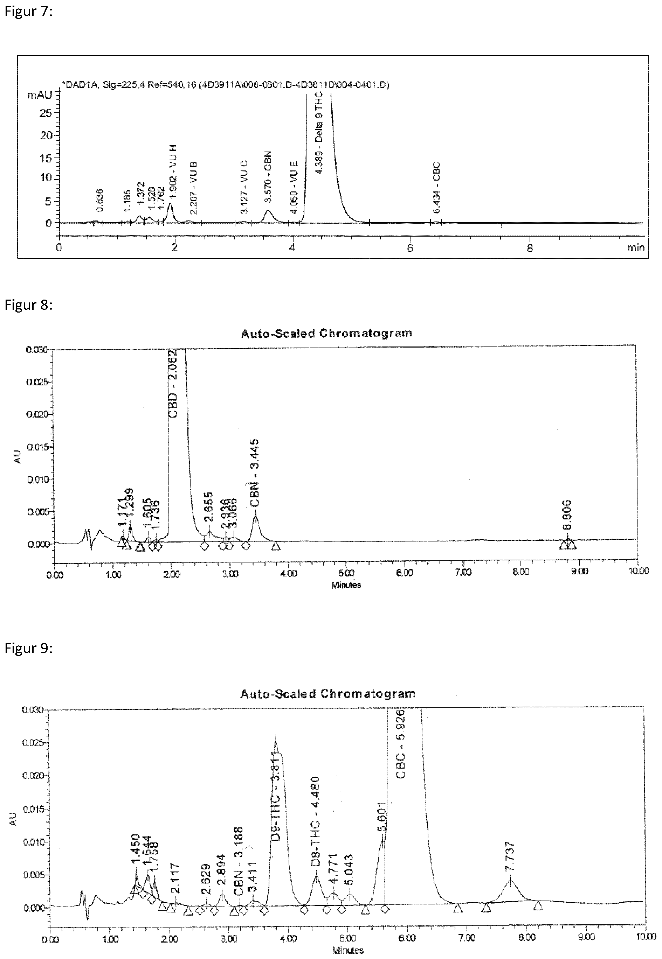

Gewinnung von THC (Dronabinol) (Figur 1-7), CBD (Figur 8) oder CBC (Figur 9) aus Drogenhanf oder Nutz-,Faserhanf: Extraktion Cannabis flos mittels Heptan, Decarboxylierung bei 120°C im Vakuum, Auflösen in Heptan, Aufreinigung in der CPC.

### 1. CPC

1. Lauf: quantitative Abtrennung CBN, anteilige Abtrennung CBC:
   Fließmittel: Acetonitril TBME ca. 12%
   Stationäre Phase: Heptan TBME ca. 10%
   aus Dronabinol 84,1% mit ca. 15,9% Verunreinigungen, wobei 5,4% CBN und 2,2% CBC sind.

Farbe: tiefbraun bis schwärzlich.
*Figur 1*
   Es entsteht:
   Dronabinol 97,7% mit ca. 2,3% Verunreinigungen, wobei 0,4% CBN und 0,9% CBC sind.
   Farbe: gelb.
*Figur 2*
   Optional 2. Lauf: quantitatve Abreinigung von CBC
   Fließmittel: Acetonitril TBME ca. 12%
   Stationäre Phase: Heptan TBME ca. 10%
   aus Dronabinol 97,7% mit ca. 2,3% Verunreinigungen, wobei 0,8% CBN und 0,9% CBC sind.
   Farbe: gelb.
*Figur 3*
   Es entsteht:
   Dronabinol 99,6% mit ca. 0,4 % Verunreinigungen, wobei 0,04% CBN und 0,05% CBC sind.
   Farbe: gelblich (weitere Farbaufhellung)
*Figur 4*
   Referenzbeispiel 2: CPC mit nachgelagertem Lauf über Kieselgelsäule
   Lauf: quantitative Abtrennung CBN
   keine Abtrennung CBC
   Fließmittel: Acetonitril purum
   Stationäre Phase: Heptan purum
   aus Dronabinol 81% mit ca. 19% Verunreinigungen, wobei 4,2% CBN und 1,6% CBC sind.
   Farbe schwärzlich bis tiefbraun
*Figur 5*
   Es entsteht:
   Dronabinol 94,5% mit ca. 5,5% Verunreinigungen, wobei 0,16% CBN und 1,4% CBC sind.
   Farbe: gelb.
*Figur 6*
   Anschließend präparative Chromatographie über Kieselgel zur quantitativen Abtrennung von CBC:
   Fließmittel Heptan 3% TBME
   aus Dronabinol 96,4% mit ca. 3,6% Verunreinigungen, wobei 0,36% CBN und 1,8% CBC sind.
   Farbe: gelb.
*Figur 7*
   Es entsteht:
   Dronabinol 98,6% mit ca. 1,4% Verunreinigungen, wobei 0,4% CBN und 0,0% CBC sind.
   Farbe: farblos.
*Figur 8**:*
   Es entsteht:
   Cannabidiol 99,3% mit ca. 0,7% Verunreinigungen, wobei 0,3% CBN ist.
   Farbe: farblos, kristallisierbar
*Figur 9*
   Es entsteht:
   Cannabichromen 94,1% mit ca. 5.9% Verunreinigungen, wobei Dronabinol 3,39% und Δ8-THC 0,19% sind.

## Patentansprüche

1. Verfahren zur Trennung und/oder Reinigung von Cannabinoiden aus einem Cannabis Extrakt, das Verfahren enthaltend mindestens einen Flüssig-flüssig-Verteilungschromatographieschritt bestehend aus einer ersten Lösungsmittelphase und einer zweiten nicht mischbaren flüssigen Phase, wobei die erste Lösungsmittelphase Cyclohexan, Heptan oder Octan ist, das durch Zentrifugalkraft stationär gehalten wird und 1% bis 15% (v/v) t-Butylmethylether der ersten Lösungsmittelphase und/oder der zweiten nicht mischbaren flüssigen Phase zugesetzt wird.

2. Verfahren zur Trennung und/oder Reinigung von Cannabinoiden aus einem Cannabis Extrakt nach Anspruch 1, wobei die erste Lösungsmittelphase n-Heptan ist.

3. Verfahren zur Trennung und/oder Reinigung von Cannabinoiden aus einem Cannabis Extrakt nach Anspruch 1 oder 2, wobei die zweite nicht mischbare flüssige Phase Acetonitril, Methanol, Ethylacetat oder Wasser ist.

4. Verfahren zur Trennung und/oder Reinigung von Cannabinoiden aus einem Cannabis Extrakt nach einem der Ansprüch 1 bis 3, wobei die zweite nicht mischbare flüssige Phase Acetonitril ist.

5. Verfahren zur Trennung und/oder Reinigung von Cannabinoiden aus einem Cannabis Extrakt nach Anspruch 1, wobei die erste Lösungsmittelphase Heptan ist und die zweite nicht mischbare flüssige Phase Acetonitril ist.

6. Verfahren zur Trennung und/oder Reinigung von Cannabinoiden aus einem Cannabis Extrakt nach einem der Ansprüche 1 bis 5, wobei die erste Lösungsmittelphase und/oder die zweite nicht mischbare flüssige Phase mit 9 bis 13 % (v/v) t-Butlymethylether versetzt ist.

7. Verfahren zur Trennung und/oder Reinigung von Cannabinoiden aus einem Cannabis Extrakt nach einem der Ansprüche 1 bis 6, mit einem Fluss von 50 mL bis 600 mL pro Minute und/oder einer Umdrehungszahl von 50 rpm bis 1.500 rpm.

8. Verfahren zur Trennung und/oder Reinigung von Cannabinoiden aus einem Cannabis Extrakt nach einem der Ansprüche 1 bis 7, wobei die erste Lösungsmittelphase und die zweite nicht mischbare flüssige Phasen im Gegenstrom geführt werden und eine kontinuierliche Abtrennung erfolgt.

9. Verfahren zur Trennung und/oder Reinigung von Cannabinoiden aus einem Cannabis Extrakt nach einem der Ansprüche 1 bis 8, wobei mindestens eine präparative Säule vor- oder nachgeschaltet ist.

10. Verfahren zur Trennung und/oder Reinigung von Cannabinoiden aus einem Cannabis Extrakt nach einem der Ansprüche 1 bis 9, wobei ein Zentrifugal-Verteilung Chromatograph verwendet wird.

## Claims

1. A method for separating and/or purifying cannabinoids from a cannabis extract, the method including at least one liquid-liquid partition chromatography step comprising a first solvent phase and a second non-miscible liquid phase, wherein the first solvent phase is cyclohexane, heptane, or octane and is kept stationary by centrifugal force, and 1% to 15% (v/v) t-butyl methyl ether is added to the first solvent phase and/or the second non-miscible liquid phase.

2. The method for separating and/or purifying cannabinoids from a cannabis extract according to claim 1, wherein the first solvent phase is n-heptane.

3. The method for separating and/or purifying cannabinoids from a cannabis extract according to claim 1 or 2, wherein the second non-miscible liquid phase is acetonitrile, methanol, ethylacetate or water.

4. The method for separating and/or purifying cannabinoids from a cannabis extract according to any one of claims 1 to 3, wherein the second non-miscible liquid phase is acetonitrile.

5. The method for separating and/or purifying cannabinoids from a cannabis extract according claim 1, wherein the first solvent phase is heptane and the second non-miscible liquid phase is acetonitrile.

6. The method for separating and/or purifying cannabinoids from a cannabis extract according to any one of claims 1 to 5, wherein 9 to 13% (v/v) t-butyl methyl ether is added to the first solvent phase and/or the second non-miscible liquid phase.

7. The method for separating and/or purifying cannabinoids from a cannabis extract according to any one of claims 1 to 6, having a flow of 50 mL to 600 mL per minute and/or a speed of 50 rpm to 1,500 rpm.

8. The method for separating and/or purifying cannabinoids from a cannabis extract according to any one of claims 1 to 7, wherein the first solvent phase and the second non-miscible liquid phase are conducted in the counter current and continuous separation occurs.

9. The method for separating and/or purifying cannabinoids from a cannabis extract according to any one of claims 1 to 8, wherein at least one preparative column is provided upstream or downstream.

10. The method for separating and/or purifying cannabinoids from a cannabis extract according to any one of claims 1 to 9, wherein a centrifugal partition chromatograph is used.

## Revendications

1. Procédé de séparation et/ou de purification de cannabinoïdes à partir d'un extrait de cannabis, ce procédé comprenant au moins une étape de chromatographie de partage liquide-liquide constituée d'une première phase de solvant et d'une deuxième phase liquide non miscible, dans lequel la première phase de solvant est du cyclohexane, de l'heptane ou de l'octane, qui est maintenue stationnaire par une force centrifuge et 1 % à 15 % (en volume) de t-butylméthyléther est ajouté à la première phase de solvant et/ou à la deuxième phase liquide non miscible.

2. Procédé de séparation et/ou de purification de cannabinoïdes à partir d'un extrait de cannabis selon la revendication 1, dans lequel la première phase de solvant est du n-heptane.

3. Procédé de séparation et/ou de purification de cannabinoïdes à partir d'un extrait de cannabis selon la revendication 1 ou 2, dans lequel la deuxième phase liquide non miscible est de l'acétonitrile, du méthanol, de l'acétate d'éthyle ou de l'eau.

4. Procédé de séparation et/ou de purification de cannabinoïdes à partir d'un extrait de cannabis selon l'une des revendications 1 à 3, dans lequel la deuxième phase liquide non miscible est de l'acétonitrile.

5. Procédé de séparation et/ou de purification de cannabinoïdes à partir d'un extrait de cannabis selon la revendication 1, dans lequel la première phase de solvant est de l'heptane et la deuxième phase liquide non miscible est de l'acétonitrile.

6. Procédé de séparation et/ou de purification de cannabinoïdes à partir d'un extrait de cannabis selon l'une des revendications 1 à 5, dans lequel la première phase de solvant et/ou la deuxième phase liquide non miscible est mélangée avec 9 à 13 % (en volume) de t-butylméthyléther.

7. Procédé de séparation et/ou de purification de cannabinoïdes à partir d'un extrait de cannabis selon l'une des revendications 1 à 6, avec un débit de 50 ml à 600 ml par minute et/ou une vitesse de rotation de 50 rpm à 1500 rpm.

8. Procédé de séparation et/ou de purification de cannabinoïdes à partir d'un extrait de cannabis selon l'une des revendications 1 à 7, dans lequel la première phase de solvant et la deuxième phase liquide non miscible sont guidées à contre-courant et une extraction continue est effectuée.

9. Procédé de séparation et/ou de purification de cannabinoïdes à partir d'un extrait de cannabis selon l'une des revendications 1 à 8, dans lequel au moins une colonne préparative est disposée en amont ou en aval.

10. Procédé de séparation et/ou de purification de cannabinoïdes à partir d'un extrait de cannabis selon l'une des revendications 1 à 9, dans lequel un chromatographe de partage centrifuge est utilisé.
